# EUROPEAN PATENT APPLICATION

(11) **EP 1 972 335 A1**
(43) Date of publication of application: **24.09.2008**
(21) Application number: 07006055.3
(22) Date of filing: 23.03.2007
(51) Int. Cl.: A61K 9/16, A61K 9/20, A61K 31/165

(54) **Solid dosage forms comprising aliskiren and pharmaceutically acceptable salts thereof**

(71) Applicant: KRKA, 8501 Novo mesto (SI)
(72) Inventor: Vrecer, Franc, 8351 Straza pri Novem mestu (SI); Skrabanja, Vida, 8000 Novo mesto (SI); Zajc, Natalija, 8340 Crnomelj (SI)
(74) Representative: UEXKÜLL & STOLBERG

(57) **Abstract**

The present invention relates to a pharmaceutical formulation comprising aliskiren or a pharmaceutically acceptable salt thereof as the active ingredient, wherein the parmaceutical formulation is present in a solid dosage form suitable for oral administration based on a granulate obtained by a hotmelt and solvent-free granulation process, and to a process for manufacturing a pharmaceutical formulation.

## Description

### Field of the Invention

The present invention relates to stable pharmaceutical compositions comprising a non-peptide orally effective renin inhibitor in a solid dosage form, and to processes for preparing such compositions. In particular, the present invention relates to dry granulation techniques using meltable excipients for preparing solid dosage forms of aliskiren or pharmaceutically acceptable salts thereof.

### Background of the Invention

Aliskiren is the first in a new class of potent, orally effective renin inhibitors for the treatment of hypertension and the hemifumarate salt thereof is registered under the tradename Tekturna® by Novartis Pharmaceutical Corporation. Chemically it is defined as (2S,4S,5S,7S)-5-amino-N-(2-carbamoyl-2-methylpropyl)-4-hydroxy-2-isopropyl-7-[4-rnethoxy-3-(3-methoxypropoxy)-benzyl]-8-methylnonanamide hemifumarate. Aliskiren in the form of free base is represented by the following formula: 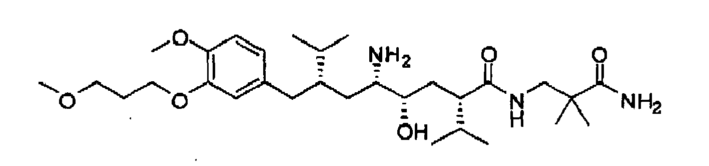 Aliskiren, in particular its hemifumarate salt is disclosed in EP-A-0 678 503. The multistage synthesis known from EP-A-0 678 503 was further improved in terms of the number of process steps, yield and industrial applicability as described in e.g. EP-A-1 303 478, EP-A-1 215 201, Tetrahedron Letters 2000, 41 (51), 10085-10089, Tetrahedron Letters 2000, 41 (51), 10091-10094, Tetrahedron Letters 2005, 46 (37), 6337-6340, WO 02/02508, and WO 2006/024501.

Clinically, aliskiren targets the renin-angiotensin system (RAS) at the first and rate-limiting step by inhibiting renin in the synthesis of angiotensin I and II which results in reduced plasma renin activity. The mechanism and the ability of aliskiren for the treatment of hypertension as monotherapy is therefore in contrary to the mechanisms known for angiotensin converting enzyme inhibitors (ACE inhibitors) and angiotensin receptor blockers (ARBs). The synergistic effects of aliskiren in combination with ACE inhibitors, ARBs, antidiabetics or diuretics are described in e.g. EP-A-1 341 533, EP-A-1 602 370, EP-A-1 507 558, US 2003/0114389, WO 03/099279, WO 2005/070406, and WO 2005/077418.

The physico-chemical characteristics render aliskiren difficult to formulate into a stable dosage form for oral administration. In particular, the technological characteristics of aliskiren are non-optimal for direct compression of powdered forms into tablets.

For example, WO 2005/089729 describes aliskiren as a highly hygroscopic material with a needle shaped crystal habit, which has a negative influence on the bulk properties of the drug substance, particle size distribution, bulk density, flowability, wetting behavior, surface area and sticking tendency. The poor compression behavior, strong elastic component and high dose of aliskiren are other hurdles in the formulation of a stable solid dosage form. It is also known from WO 2005/089729 that water-based wet granulation procedures can not be used since aliskiren changes to an amorphous state on its contact with water, which results in decreased stability of the product. On the other hand, WO 2005/089729 teaches that neither direct compression nor roller compaction can be used for routine production of a high quality final product. In view of such problems the authors of WO 2005/089729 suggest a process for preparing a formulation of aliskiren based on wet granulation of aliskiren using a mixture of organic solvents or a binder solution in organic solvents. The drawback of such a process is that residual solvents and impurities in the product of such granulation may render the resulting formulation unstable and that drying steps are needed.

In solid dosage forms, surfactants have been widely shown to enhance drug dissolution rate. This may be due to wetting effects resulting in increased surface area, effects on solubility and effective diffusion coefficients, or a combination of these effects. The prior art relating to aliskiren describes the use of anionic surfactants such as organic acids, and their physiologically tolerated salts of alkali metals or alkaline earth metals, which contain a hydrophobic substituent.

For example, EP-A-1 517 682 describes renin inhibitors, preferably aliskiren, as relatively large molecules which are very readily soluble in water but have properties that suggest low oral bioavailability. Adding anion surfactants to compositions containing said renin inhibitors is disclosed to increase oral bioavailability.

Thus, the specific physical and chemical properties and the high doses of aliskiren have been known to cause problems in the preparation of formulations suitable for an effective oral administration. Hence, a need exists for formulations of aliskiren for effective oral delivery, which formulations provide for an appropriate disintegration time and/or appropriate solubility and/or dissolution profile of the active principle. In addition, it is desirable to be able to prepare such formulations by using an economical process which avoids the problems of the prior art as noted above, e.g. a process without the need for drying steps and without the concern associated with the presence of residual solvents in the formulation resulting from process, e.g. granulates and/or tablets.

### Summary of the invention

In accordance with the present invention there is provided a pharmaceutical formulation comprising aliskiren or a pharmaceutically acceptable salt thereof as the active ingredient, wherein the pharmaceutical formulation is present in a solid dosage form suitable for oral administration based on a granulate obtained by a hot-melt and solvent-free granulation process.

In another embodiment of the present invention, there is provided a process for the manufacture of a pharmaceutical formulation comprising:
a) granulating aliskiren or a pharmaceutical salt thereof alone or in admixture with one or more excipients selected from binders, fillers, disintegrants, wetting agents and/or lubricants at elevated temperature and in the essential absence of solvents;
b) optionally formulating the granulate obtained in step (a) into tablets, film-coated tablets, pills, lozanges, sachets, soft or hard gelatine capsules.

The present invention also provides a pharmaceutical formulation as defined above for the treatment of hypertension, congestive heart failure, angina, myocardial infarction, artherosclerosis, diabetic nephropathy, diabetic cardiac myopathy, renal insufficiency, peripheral vascular disease, left ventricular hypertrophy, cognitive dysfunction, stroke, headache and chronic heart failure.

Further preferred embodiments of the present invention are disclosed in the dependent claims attached to this description.

Thus, according to the present invention heat is utilized during a process of preparing a pharmaceutical formulation comprising a granulate containing aliskiren or a pharmaceutically acceptable salt thereof to solve the above mentioned problems, including the above-mentioned stability issue associated with wet granulation techniques. The granulation process of the present invention is referred to herein as "hot-melt granulation", i.e. granulation at elevated temperature. More specifically, the temperature used in the granulation process may be in the range of 40 to 90°C, e.g. 50 to 70 °C, preferably 45 to 65 °C.

Hot-melt granulation means any granulation process where the drug and optional excipients are granulated with binder which is in a molten state. Binder's melt can be obtained either by heating all components including the binder in the bowl of the granulating equipment such as high shear mixer to the temperature of at least the melting point of the binder or by melting the binder in a mixture with other components by the heat formed by friction of particles while mixing in the bowl of a mixer or by the pressure during the compaction of drug. In a special embodiment of the invention, hot-melt granulation can be carried out by spraying the molten binder onto moving particles of other components of the granulate including aliskiren or a salt thereof.

Furthermore, the process of the present invention is based on a granulation technique which is solvent-free so that there is no need for drying steps in the preparation of the granulate and in particular no organic solvents are used in the process. By "solvent-free" is meant that the granulation process of the present invention is conducted in the essential absence of water and other solvents, in particular organic solvents such as those used in the prior art discussed hereinabove.

Preferably, the active ingredient is at least one pharmaceutically acceptable salt of aliskiren, and most preferably aliskiren hemifumarate. Optionally, a diuretic, preferably hydrochlorothiazide (HCTZ) or indapamide is also included in the formulation.

### Detailed Description of the Invention

In the following description, any reference to the term "aliskiren" is intended to include the pharmaceutically acceptable salts thereof, and especially aliskiren hemifumarate.

The formulation of the invention is an oral solid dosage form based on a granulate obtained from a hot-melt, solvent-free granulation process. The granulate obtained by the process of the present invention can be further formulated into tablets, film coated tablets, pills, lozenges, sachets, soft and hard gelatin capsules.

The formulations of the invention are preferably provided in a solid unit dosage form, each dosage containing about 1-600 mg, more preferably about 75-600 mg, and even more preferably about 150-300 mg of aliskiren in its free form, i.e. as base. In case the hemifumarate salt of aliskiren is used, a therapeutic dose of 75 mg, 150 mg, 300 mg and 600 mg of free aliskiren corresponds to 82.88 mg, 165.75 mg, 331.50 mg and 663.00 mg of aliskiren hemifumarate, respectively.

The term "unit dosage form" refers to physically discrete units suitable as unitary dosages for humans and other mammals, each containing a predetermined quantity of aliskiren calculated to produce the desired therapeutic effect, in association with a suitable pharmaceutical excipient.

In the following description of the process for preparing the pharmaceutical formulation of the present invention, reference is made to intragranular excipients and extragranular excipients. The excipients used during the preparation of the granulate are referred to herein as intragranular excipients, while the excipients which are added during the further processing of the granulate into dosage forms such as tablets are referred to as extragranular excipients.

The invention is based on solid dosage forms containing agglomerated aliskiren particles, which as such have inappropriate physical properties such as flowability, compressibility and physical stability, which particles are obtained by a solvent-free granulation process using one or more binders characterized by a melting point which is at least 10 °C, preferably 20 °C and more preferably at least 25 °C lower than the melting point of aliskiren or its salt, optionally in combination with one or more intragranular exicipients selected from fillers, disintegrants, wetting agents and/or lubricants.

In a special embodiment of the invention where aliskiren hemifumarate is used as the active drug, the selected binders are characterized by having a melting point in the range between 40 to 90°C, preferably between 45 to 65 °C.

Typical binders for use in the present invention can be selected from the group of polyethylenglycols with molecular weights of 1,500 to 20,000 such as PEG 4,000, PEG 6,000, poloxamers with molecular weights in the range of 5,000 to 20,000 such as Poloxamer 188, Poloxamer 237, Poloxamer 338 or Poloxamer 407 commercialy available as Pluronic^{®} or Lutrol^{®} types, C₈-C₁₈ fatty acid esters of polyoxyglicerides of Gelucire^{®} type, C₈-C₁₈ fatty acid esters of polyethylene glycole, esters of glycerol with organic carboxylic acid such as glycerol mono and distearate, glycerol behenate, sugar esters such as sucrose stearate, sucrose palmitate or sucrose laurate.

The mass ratio between the drug and a binder in the granulate is in the range 9.5:0.5 to 1:1, preferably 9:1 to 2:1.

As an intragranular filler, spray-dried lactose, microcrystalline cellulose (MCC), corn or potato starch, pregelatinized starch, mannitol may be used in an amount of 5-40 w/w %, preferably 10-30 w/w % calculated on the total composition of granulate.

Intragranular disintegrants comprise crospovidone, crosslinked carboxymethylcellulose, sodium carboxymethylstarch, low substituted hydroxypropylcellulose (L-HPC) in an amount of 0.5-20 w/w %, preferably 1-10 w/w % calculated on the total composition of granulate. Optionally, a disintegrant with an average particle size smaller than 80. µm can be used in the inner phase of granulate. The preferred disintegrant in such a case is crospovidone with average particle size 10-60 µm, especially preferred is the crospovidone type Polyplasdone^{®} XL10 produced by ISP.

Wetting agents for intragranular use comprise sodium alkyl sulphates such as sodium lauryl sulphate, sorbitan derivatives such as polysorbate 80, polysorbate 60 or a combination thereof in an amount of 0.5-10 w/w %, preferably 1-5 w/w %, based on the total composition of granulate.

As an intragranular lubricant, talc, metal stearates such as magnesium, calcium, sodium, aluminium or zinc stearate can be used in an amount of 0.1-5 w/w %, preferably 0.2-2 w/w % calculated on the total composition of granulate.

Aliskiren can be incorporated into the granulate in the form of the free base or pharmaceutically acceptable salt in a weight fraction of 30-90 w/w%, preferably 35-80 w/w%, based on the total weight of the granulate. The hemifumarate salt of aliskiren is preferred.

Mixing of the excipient(s) with aliskiren may be effected in a conventional device used for mixing of powders, e.g. a motionless (passive) mixer, fluidized bed mixer, diffusion mixer, biconic diffusion mixer, uniconic mixer, biconic mixer, turbular mixer, cubic mixer, planetary mixer, Y-shaped mixer, v-shaped mixer and preferably high-shear mixer.

The agglomeration (granulation) step is performed in the essential absence of solvents. In one of the embodiments of the invention, granulation is performed by roller compaction of a mixture of aliskiren or its pharmaceutically acceptable salt such as hemifumarate salt with binder with melting point in the range of 40 to 90°C, preferably 45-65 °C and optional other excipients selected from intragranular fillers, disintegrants, wetting agents and/or lubricants.

Pharmaceutical formulations of the present invention can comprise any crystal or amorphous form of aliskiren and/or any crystal or amorphous form of salts thereof including hydrates and/or solvates.

The oral dosage form of the present invention, can also be formulated with aliskiren in the form of needle shaped crystals, wherein the ratio of crystal length : crystal width may be about 10:1 1 or larger, or with crystals having a length : width ratio of from about 1:1 to <10:1, preferably from about 1:1 to 5:1.

Final crystals of aliskiren or salts thereof can be obtained by techniques well known in the art, such as milling, crystallization, suspending or different ways of drying using each referred step alone or in combination with each other. Milling or processing in any other way which can further reduces particle size and/or change the ratio of the crystal dimensions can be selected and used by a person skilled in the art. For the milling purposes air jet mill, ball mill or hammer mill are commonly used as milling equipment. An average particle size of active drug used in the present pharmaceutical formulation is in the range between 0.2 µm and 400 µm, preferably between 5 µm and 300 µm and most preferably between 10 µm and 150 µm.

The average particle size is determined with a Malvern Mastersizer MS 2000 instrument. Usually, 100-800 mg of substance are dispersed in 5-8 ml of dispersant. According to manufacturer's information, the Malvern Mastersizer allows for a measurement of particle size distributions within the range of 20 nm to 2000 µm, with a precision of better than 0.5%. Particle sizes are determined by measuring the angular distribution of laser light scattered by a homogeneous suspension of particles. The size distribution is determined from the light scattering data using the theory of light scattering developed by Gustav Mie.

In another embodiment of the invention, aliskiren or a pharmaceuticaly acceptable salt thereof such as the hemifumarate salt is mixed with other intragranular excipients, where the binder with melting point in the range of 40 to 90°C, preferably 45-65 °C is added directly to the mixture of drug and excipients or is added in melted form during the granulation.

The granulation process can be performed either by *in-situ* melting of binder in a granulation bowl such as a high shear mixer, or by spraying melted binder on the mixture of drug and excipients in granulation equipment such as a high shear mixer or fluid bed granulator adjusted for hot-melt granulation. After granulation, the cooled mass is sieved through a sieve with mesh opening size of 0.5 to 2 mm, preferably 0.71 to 1.4 mm and mixed with extragranular excipients.

In a special embodiment of the invention, aliskiren or a pharmaceuticaly acceptable salt thereof can be granulated by a hot-melt extrusion process, wherein the mixture of drug, binder having a melting point in the range of 40 to 90 °C, preferably 45-65 °C and optional other excipients selected from intragranular fillers, disintegrants, wetting agents and/or lubricants is heated to at least the melting temperature of a binder and extruded. Obtained extrudates are being cut and optionally spheronized into spherical granules, which can be further coated by a film coating and later filled into capsules or sachets or compressed into tablet cores.

The granulate is incorporated into solid dosage forms such as tablets, film coated tablets or capsules in amount of 20-99 w/w %, preferably 40 to 90 w/w % based on the total weight of solid dosage form.

The solid dosage forms of the present invention can further comprise at least one extragranular excipient. Extragranular excipients can be selected from fillers such as starches like corn starch or pregelatinized starch, compressible sugar, dextrin, dextrose, powdered cellulose, microcrystalline cellulose, silicified microcrystalline cellulose, lactose in a monohydrate or spray dried form, direct compressible mannitol, calcium hydrogen phosphate, disintegrants such as sodium or calcium carboxymethylcellulose, crospovidone, crosslinked carboxymethyl cellulose, low substituted hydroxypropyl cellulose, alginic acid, sodium alginate, glidants such as colloidal silicon dioxide, talc, magnesium trisilicate and lubricants such as metal stearates comprising magnesium, calcium, sodium, aluminium or zinc stearate, hydrogenated castor oil, talc, sodium stearyl fumarate, stearic acid, polyethylene glycols (PEG) with average molecular weight higher than 3000, i.e. PEG 6000, PEG 8000.

The ratio of intra and extragranular amounts of disintegrators can be in the range of 2:1 to 1:9, preferably 1:1 to 1:5. Combinations of intra and/or extragranular disintegrators can also be used.

It has been found that compressibility can be further improved by using granulated microcrystalline cellulose (MCC) in the outer phase (extragranularly) as filler. Granulated microcrystalline cellulose can be obtained either by a process of extrusion spheronization using PEG, such as PEG 4000, as a binder, wherein the ratio of PEG:MCC of 2:1 to 1:9, or by wet granulation of MCC with aqueous solutions of water soluble polymers such as methylcellulose (MC), hydroxyethylcellulose (HEC), hydroxypropylcellulose (HPC), hydroxypropylmethylcellulose (HPMC) or povidone (PVP) as binders in the ratio of binder:MCC of 1:4 to 0.25:9.75

The granulate containing aliskiren or a pharmaceutically acceptable salt thereof, which granulate may be obtained by one of the above-mentioned methods can be formulated into capsules or tablets directly or after mixing with extragranular excipients (in accordance with procedures well-known to one of ordinary skill in the the field of pharmaceutical technology). Tablet cores can be further coated with a coating containing one or more water soluble polymers selected from cellulose ether, polyvynil alcohol, methacrylate copolymers such as low viscosity types (with viscosity of 2 % water solution 2-20 cp) of HPMC, polyvinyl alcohol (PVA), hydroxypropylcellulose (HPC), aminoalkyl methacrylate copolymer (Eudragit^{®}E), antitacking agent selected from talc or glycerol monostearates, pigments such as metal oxides. Preferred are coatings with lower water vapour permeability such as those based on PVA or aminoalkyl methacrylate copolymer.

The concentration of aliskiren in the form of its free base or its pharmaceutically acceptable salt in the final dosage form such as tablet or capsule can be in the range 20 to 90 w/w %, preferably 30 to 60 w/w %.

As the presence of water can influence physical transformation of crystalline aliskiren into the amorphous state resulting in decreased chemical stability, the use of excipients in intra and extragranular phases with low moisture content is preferred.

In order to achieve optimal functionality and to ease the swallowing of the tablets by patients, especially older ones, coating of the tablets is preferred and appropriate tablet shape, preferably oblong is selected such that the length to width ratio is between 1.5 and 3, preferably between 1.8 and 2.6.

The size of tablets is defined also by the tablets' weight. In order to optimize their size, the weight of tablets should be in the range of 100-250 mg, 200-500 mg and 400-1000 mg containing 75, 150 and 300 mg, respectively, of aliskiren.

In the process of the invention as described above, the compression of granulate, in particular to cores/tablets, can be effected using an automatic rotary compressing machine from different manufacturers of equipment for use in pharmaceutical industry. Aliskiren formulations can further contain a diuretic such as hydrochlorothiazide or indapamide. Hydrochlorothiazide or indapamide, if added, can be present exclusively within the granulate, or they can be added exclusively as extragranular phase after the granulation, or they can be divided between the granulate and extragranular phases. The optional additional amount of hydrochlorothiazide in these formulations is preferably 5-50 mg, preferably 10-30 mg per dosing unit. Any combinations of these amounts are possible, i.e. the ratio of aliskiren (preferably aliskiren hemifumarate) to hydrochlorothiazide (mg:mg) may preferably be 75:12.5, 75:25, 150:12.5, 150:25, 300:12.5, 300:25, 600:12.5, and 600:25. In case indapamide is used as a diuretic in fixed combinations, 0.5 to 3 mg, preferably 1.25 mg of indapamide is combined with 75, 150, 300 or 600 mg aliskiren (free base).

The pharmaceutical formulations of the present invention may also comprise aliskiren or pharmaceutically acceptable salts thereof in combination with at least one therapeutic agent selected from the group of antihypertensives, lipid regulators and antidiabetics.

A compound from the group of antihypertensives may be selected from the group consisting of angiotensin converting enzyme (ACE) inhibitors, angiotensin receptor blockers (ARBs), AT₁-receptor antagonists, calcium-channel blockers (CCBs) and antiadrenergics.

For example, ACE inhibitors may be selected from the group consisting of captopril, enalapril, lisinopril, trandolapril, cilazapril, ramipril, fosinopril, perindopril or a pharmaceutically acceptable salt thereof.

AT₁-receptor antagonist for use in the combined formulation may be selected from the group consisting of candesartan, irbesartan, losartan, olmesartan, telmisartan, valsartan or pharmaceutically acceptable salts thereof.

The calcium channel blocker may be selected from the group consisting of amlodipine, diltiazem, felodipine, nifedipine, nitrendipine and verapamil and salts thereof.

From the group of β-adrenergic blockers compounds like acebutol, atenolol betaxolol, bisoprolol, metoprolol, and pharmaceutically acceptable salts can be selected. As mixed α-and β-adrenergic blockers carvedilol may be included in the formulation.

Lipid regulators for combination with aliskiren in the formulation of the present invention can be selected from of 3-hydroxy-3-methylglutaryl coenzyme A (HMG CoA) reductase inhibitors such as lovastatin, simvastatin, pravastatin, atorvastatin, fluvastatin, cerivastatin, rosuvastatin, and salts thereof.

The antidiabetic agent may be selected from the group of sulfonyl urea, meglitinides (such as nateglinide, repaglinide) and pharmaceutically acceptable salts thereof, thiazolidinediones (such as pioglitazone, rosiglitazone) and pharmaceutically acceptable salts thereof, alpha glucosidase inhibitors, incretin mimetics, or biguanides such as metformin or the like and the pharmaceutically acceptable salts thereof.

Colorants/opacifiers for use in the present invention include organic dyes and their lakes, inorganic colors and natural colors, including water soluble colors and water-insoluble colors (pigments).

Different materials from each group can also be combined in defined ratios. Film coating suspensions can also be used as ready-to-use preparations, which are commercially available.

Film coating dispersions can be prepared by using different vehicles (solvents, such as water, alcohols, ketones, esters, chlorinated hydrocarbons and mixtures thereof. A preferred solvent is water.

Particularly preferred is a composition of the coating suspension comprising, in terms of wt.-% based on the dry material, 1-99 % (preferably 1-95 %) polymer, 1-30 % (preferably 1-7.0 %) plasticizer, 2-30 % (preferably 5-25 %) antitacking agent and 0.1-20 % (preferably 0.1-10 %) colorant/opacifier.

Conventional equipment for applying a coating, such as a Wurster coating system or conventional coating pans can be used.

Any pharmaceutical composition may be prepared and stored in a packaging material usually chosen by those of ordinary skill in the art of maintaining stability of active drugs. Decreased moisture permeability of primary packaging material is preferred in order to diminish moisture sorption by aliskiren containing dosage forms to avoid any changes in drug stability. Low moisture permeable primary packaging materials such as aluminium or polychloro-3-fluoroethylene homopolymer/PVC laminate can be used with the thickness in the range 10 to 40 µm in case of Al/Al blisters and 10 to 110 µm in case of Al-polychloro-3-fluoroethylene homopolymer/PVC laminate blisters. Optionally, dosage forms containing aliskiren or its pharmaceutically acceptable salt can be packed into primary packaging with desiccant. Desiccant can be placed inside the packaging unit together with aliskiren dosage units such as tablets and/or in the closure system or can be incorporated into the walls of the primary packaging unit.

According to a preferred embodiment, contact between the pharmaceutical composition and environmental oxygen may be reduced or suppressed by either packaging the pharmaceutical composition under reduced pressure, packaging in an inert gas atmosphere, using a coating affording protection and stability of the pharmaceutical composition to environmental influences, or by using a packaging wherein the contact between the pharmaceutical composition and oxygen is reduced by the means of oxygen absorbers.

An atmosphere with reduced oxygen content or reduced oxygen partial pressure may be obtained by the use of an atmosphere of reduced pressure, e.g. by creating a partial vacuum by means of a suitable pump or by partial freezing or liquefying the atmosphere, by the use of an inert gas atmosphere, wherein as an inert gas nitrogen or argon may be used, or by the use of absorbents. Suitable absorbents may be selected from the group of commercially available absorbents such as humidity-activated oxygen absorbers, ultraviolet-radiation-activated absorbers, radiation-activated absorbers, microwaves-radiation-activated absorbers, absorbers activated by a combination of activation processes or absorbers without necessity of activation. Examples of commercially available absorbers are Ageless^{™} (Mitsubishi Gas Chemical), ATCO (Standa Industry), FreshPax^{™} (Multisorb Technologies), O-Buster^{™} (Hsiao Sung Non-Oxygen Chemical Co), Biotika Oxygen Absorber (Biotika) and the like.

The invention also provides a stabilized package of aliskiren which is provided with a space for trapping and disposal of free oxygen. Moreover, if the active compounds of the present composition are exhibited to a reduced oxygen partial pressure, the formulation is preferably enclosed in a substantially gas exchange non-permeable material and an atmosphere with reduced oxygen partial pressure is contained in the packaging. The substantially gas exchange non-permeable package is preferably selected from the group consisting of an Al/Al blister, an Al-polychloro-3-fluoroethylene homopolymer/ PVC laminate blister.

Aliskiren or its salt dissolves rapidly from the solid dosage forms produced according to the present invention. More than 20%, 40% and 70% of the dose is dissolved in 10, 20 and 30 minutes, respectively, as determined by using the USP Basket Method (100 rpm, 500 mL of 0.1 M hydrochloric acid, 37±0.5 °C, UV or HPLC detection). The absolute amount of excipients and their ratios is dependent on the desired properties of the solid oral dosage form and/or crystal properties of the active drug. According to the crystal properties of the given batch of the active drug and to comply with the desired dissolution profile of the active drug the absolute amount and the ratio of excipients may be defined by the person skilled in the art by routine experimentation

The following examples illustrate the invention and are not intended to restrict the scope of the invention in any way.

### Examples

**Table 1: Composition of Granulate of Examples 1-5**

| Ingredients (in %) | Ex. 1 % | Ex. 2 % | Ex. 3 % | Ex. 4 % | Ex. 5 % |
|---|---|---|---|---|---|
| Aliskiren hemifumarate | 65 | 50 | 55 | 73 | 60 |
| Macrogol 4000 (PEG 4000) | 20 | / | / | / | / |
| Poloxamer 188 (Lutrol^{®} F68) | / | 20 | / | / | / |
| Poloxamer 407 (Lutrol^{®} F127) | / | / | 15 | / | / |
| Gelucire^{®} 50/13 | / | / | / | 20 | / |
| Gelucire^{®} 44/14 | / | / | / | / | 25 |
| Polyplasdone^{®} XL10* (crospovidone) | 5 | / | / | / | / |
| Polyplasdone^{®} XL (crospovidone)** | / | 7 | 5 | 2 | 1 |
| Avicel PH101 | 10 | 23 | 25 | 5 | 14 |

| | | | | | |
|---|---|---|---|---|---|
| *... av. Particle size < 80µm **... av. Particle size > 80µm | | | | | |

The granulate was prepared according to the following steps:
- Step 1:: Aliskiren hemifumarate was mixed together with ex-cipients as stated in Table 1 in a Collette Gral 10 double jacket bowl (volume of bowl: 10 L) at room temperature for approx. 5 minutes. Total mass of powder in the bowl was 800 g.
- Step 2:: The temperature of the water in the heating/cooling jacket was increased to 3 °C above melting point of the binder material used and the mixture was mixed using appropriate impeller and chopper speed until the suitable granulate was obtained.
- Step 3:: The granulate in the bowl was cooled down to room temperature at low speed mixing by replacing the hot water in the jacket with cold tap water.
- Step 4:: The obtained granulate was milled and sieved.

**Table 2: Composition of Granulate of Examples 6-13**

| Ingredients (in %) | Ex.6 % | Ex.7 % | Ex.8 % | Ex. 9 % | Ex.1 0 % | Ex.11 % | Ex.1 2 % | Ex.13 % |
|---|---|---|---|---|---|---|---|---|
| Aliskiren hemifumarate | 60 | 60 | 57 | 55 | 55 | 50 | 63 | 65 |
| Macrogol 4000 (PEG 4000) | 10 | 10 | 10 | 20 | / | / | / | / |
| Poloxamer 188 (Lutrol^{®} F68) | / | / | / | / | 20 | / | / | / |
| Poloxamer 407 (Lutrol^{®} F127) | / | / | / | / | / | 20 | / | / |
| Gelucire^{®} 50/13 | / | / | / | / | / | / | 10 | / |
| GeLucire^{®} 44/14 | / | / | / | / | / | / | / | 10 |
| Crospovidone | / | / | / | 5 | 2 | 5 | 2 | 1 |
| Sodium carboxymethyl starch | 5 | / | / | / | / | / | / | / |
| L-HPC | / | 10 | / | / | / | / | / | / |
| Crosslinked carboxymethyl cellulose | / | / | 8 | / | / | / | / | / |
| Avicel PH105 | 25 | 20 | 25 | 20 | 23 | 25 | 25 | 24 |

The granulate was produced according to the following steps:
- Step 1:: The components according to the composition of examples 6-13 as stated in Table 2 were mixed together in Collette Gral 75 double jacket bowl (volume of bowl: 75 L) at room temperature for approx. 5 minutes. Total mass of powder in the bowl was 8 kg.

- Step 2:: The obtained mixture was compacted on a Bephex roller compactor.
- Step 3:: The compacted material was milled and sieved.

**Table 3: Composition of granulate: composition of Examples 14-16**

| Ingredients (in %) | Ex. 14 % | Ex. 15 % | Ex. 16 % |
|---|---|---|---|
| Aliskiren hemifumarate | 65 | 53 | 52 |
| Macrogol 4000 (PEG 4000)* | 20 | / | / |
| Poloxamer 407 (Lutrol^{®} F127)* | / | / | 20 |
| Gelucire^{®} 50/13* | / | 20 | / |
| Crospovidone | 5 | 2 | 3 |
| Avicel PH112 | 10 | 25 | 25 |

| | | | |
|---|---|---|---|
| * corresponding binder is added in Step 2 | | | |

The granulate was produced according to the following steps:
- Step 1:: Aliskiren hemifumarate was mixed together with the excipients as stated in Table 3 excluding the binder in Collette Gral 10 double jacket bowl (volume of the bowl: 10 L) at room temperature for approx. 5 minutes. Total mass of powder in the bowl was 800 g.
- Step 2:: The temperature of the water in heating/cooling jacket was increased to temperature of the melting point of the binder and the melted binder at melt temperature 5 °C above its melting point was sprayed into the powder mixture using appropriate impeller and chopper speed. After all binder was sprayed the mixing was continued until the suitable granulate was obtained.
- Step 3:: The granulate in the bowl was cooled down to room temperature at low speed mixing by replacing the hot water in the jacket with cold tap water.
- Step 4:: The obtained granulate was milled and sieved.

### Example 17

828.75 g of aliskiren hemifumarate, 70 g L-HPC and 250 g of Avicel PH 113 were mixed in a bag and transferred to Glatt GPCG-3 fluid bed granulator equipped with a suitable heat isolated 1.0 mm top spray nozzle. The fluidized powder mixture was granulated by spraying 300 g of Poloxamer 188 melt heated to 70 °C at an appropriate spraying rate to obtain the granulate. After all binder had been sprayed, the process was stopped and the obtained granulate was sieved.

### Examples 18-22

This example illustrates the preparation of a mixture for tableting (suitable for 2000 tablets in theory) each containing 150 mg of aliskiren in the form of free base:

| Ingredients (amounts in g) | Ex. 18 /g | Ex. 19 /g | Ex. 20 /g | Ex. 21 /g | Ex. 22 /g |
|---|---|---|---|---|---|
| Granulate from Ex. 1 | 552.5 | - | - | - | - |
| Granulate from Ex. 2 | - | 663.0 | - | - | - |
| Granulate from Ex. 8 | - | - | 581.6 | - | - |
| Granulate from Ex. 15 | - | - | - | 625.9 | - |
| Granulate from Ex. 17 | - | - | - | - | 579.5 |
| MCC (Avicel PH 112) | 150.0 | 95.0 | 120.0 | 122.5 | 100.0 |
| Pregelatinized starch | 55.5 | - | 51.4 | - | 68.5 |
| L-HPC LH11 | 30.0 | 30.0 | 35.0 | 40.0 | 40.0 |
| Colloidal silicon dioxide | 4.0 | 4.0 | 4.0 | 4.0 | 4.0 |
| Magnesium stearate | 8.0 | 8.0 | 8.0 | 8.0 | 8.0 |
| Total | 800.0 | 800.0 | 800.0 | 800.0 | 800.0 |

All components were mixed in an appropriate biconical mixer transferred to Killian Pressima 8 and pressed into 400 mg oblong tablets.

The following examples illustrate the preparation of film coated tablets containing 165.75 mg aliskiren hemifumarate (equivalent to 150 mg free base):

### Example 23:

1500 tablet cores from example 19 were coated in a laboratory perforated coating drum by a water dispersion of Opadry^{®} II HP (HPMC based) white containing 30.0 g of dry substance. The coated tablets were dried. Weight of film coated tablets is 420.0 mg.

### Example 24:

2000 tablet cores produced according to example 19 were coated in a laboratory perforated coating drum by a water dispersion of Opadry^{®} AMB (PVA based coating) white containing 50.0 g of dry substance. The coated tablets were dried. Weight of film coated tablets is 425.0 mg.

The following examples illustrate the preparation of mixtures for tableting for aliskiren tablets containing 300 mg aliskiren in the form of free base:

**Table 4: Composition of Tablet Mixtures of Examples 25 and 26**

| Ingredients | Ex. 25/ g | Ex. 26/ g |
|---|---|---|
| Granulate according to Ex. 4 | 681.2 | - |
| Granulate according to Ex. 12 | - | 789.3 |
| MCC (Avicel PH 112) | 156.8 | 300.0 |
| Spray dried lactose | 80.0 | 199.2 |
| Crospovidone | 60.0 | 69.0 |
| Colloidal silicon dioxide | 4.0 | 7.5 |
| Magnesium stearate | 8.0 | 15.0 |
| Total | 990.0 | 1380.0 |

All components were mixed in a plastic bag and compressed into oblong tablets containing 300 mg aliskiren. Average tablet weight of tablets produced according to examples 25 and 26 are 660 and 920 mg, respectively.

### Example 27

1500 tablet cores produced according to example 25 were coated in a laboratory perforated coating drum by a water dispersion of Opadry^{®} AMB (PVA based coating) white containing 60.0 g of dry substance. The coated tablets were dried. Weight of film coated tablets was 700.0 mg.

**Table 5: Composition (expressed in mg) of aliskiren film coated tablets produced according to Example 23:**

| Ingredeints | | | | |
|---|---|---|---|---|
| Strength of tablets | 75* mg | 7.50* mg | 300* mg | 600*mg |
| Aliskiren hemifumarate | 82.88 | 165.75 | 331.50 | 663.00 |
| Microcrystalline cellulose | 61.88 | 123.75 | 247.50 | 495.00 |
| Poloxamer 188 | 33.15 | 66.30 | 132.60 | 265.20 |
| Crospovidone | 11.60 | 23.20 | 46.40 | 92.80 |
| Low substituted HPC | 7.50 | 15.00 | 30.00 | 60.00 |
| Colloidal silicon dioxide | 1.00 | 2.00 | 4.00 | 8.00 |
| Magnesium stearate | 2.00 | 4.00 | 8.00 | 16.00 |
| **Total weight of tablet core** | **200.00** | **400.00** | **800.00** | **1600.00** |
| Opadry II HP | 10.00 | 20.00 | 40.00 | 80.00 |
| **Total weight of coated tablet** | **210.00** | **420.00** | **840.00** | **1680.00** |

| | | | | |
|---|---|---|---|---|
| *calculated as the corresponding amount of aliskiren free base | | | | |

## Claims

1. A pharmaceutical formulation comprising aliskiren or a pharmaceutically acceptable salt thereof as the active ingredient, wherein the pharmaceutical formulation is present in a solid dosage form suitable for oral administration based on a granulate obtained by a hot-melt and solvent-free granulation process.

2. A pharmaceutical formulation according to claim 1, wherein at least one excipient selected from the group consisting of binders, fillers, disintegrants, wetting agents and/or lubricants is present in the formulation.

3. A pharmaceutical formulation according to claim 2, wherein the at least one excipient comprises a binder having a melting point of at least 10°C lower than the melting point of the aliskiren or a pharmaceutically acceptable salt.

4. A pharmaceutical formulation according to any of the preceding claims, wherein the mass ratio between the drug and the binder in the granulate is in the range of 9.5 : 0.5 to 1 : 1.

5. A pharmaceutical formulation according to any of the preceding claims, wherein the size of the granules is 0.1 to 2 mm.

6. A pharmaceutical formulation according to any of the preceding claims, wherein aliskiren or a pharmaceutical salt thereof is used in the form of crystals.

7. A pharmaceutical formulation according to claim 6, wherein the crystals have an average particle size in the range between 0.2 µm and 400 µm.

8. A pharmaceutical formulation according to any of the preceding claims, wherein the aliskiren crystals have a ratio of crystal length to crystal width of 10 : 1 or larger; or a ratio of crystal length to crystal width of 1 : 1 to 5 : 1.

9. A pharmaceutical formulation according to any of the preceding claims, wherein a diuretic is present as additional active ingredient.

10. A pharmaceutical formulation according to any of the preceding claims, wherein as additional active ingredient one or more of an antihypertensive, a lipid regulator and an antidiabetic is present.

11. A pharmaceutical formulation according to claim 9 claims, wherein the diuretic is hydrochlorothiazide (HCTZ) or indapamide.

12. A process for the manufacture of a pharmaceutical formulation according to any of claims 1 to 11 comprising:
a) granulating aliskiren or a pharmaceutical salt thereof alone or in admixture with at least one excipient selected from binders, fillers, disintegrants, wetting agents and/or lubricants at elevated temperature and in the essential absence of solvents;
b) optionally formulating the granulate obtained in step (a) into tablets, film-coated tablets, pills, lozanges, sachets, soft or hard gelatine capsules.

13. A process for the manufacture of a pharmaceutical formulation according to claim 12, wherein the temperature used in step (a) is in the range of 40 to 90 °C.

14. A pharmaceutical formulation according to any of claims 1 to 11 or obtained by a process according to claim 12 or claim 13 for the treatment of hypertension, congestive heart failure, angina, myocardial infarction, artherosclerosis, diabetic nephropathy, diabetic cardiac myopathy, renal insufficiency, peripheral vascular disease, left ventricular hypertrophy, cognitive dysfunction, stroke, headache and chronic heart failure.

15. Use of a pharmaceutical formulation according to any of claims 1 to 11 or obtained by a process according to claim 12 or claim 13 for the manufacture of a medicament for the treatment of hypertension, congestive heart failure, angina, myocardial infarction, artherosclerosis, diabetic nephropathy, diabetic cardiac myopathy, renal insufficiency, peripheral vascular disease, left ventricular hypertrophy, cognitive dysfunction, stroke, headache and chronic heart failure.
